# EUROPEAN PATENT APPLICATION

(11) **EP 3 858 171 A1**
(43) Date of publication of application: **04.08.2021**
(21) Application number: 20154522.5
(22) Date of filing: 30.01.2020
(51) Int. Cl.: A24F 40/485, A61M 15/06

(54) **AEROSOL DELIVERY APPARATUS**

(71) Applicant: NERUDIA LIMITED, Liverpool Merseyside L24 9HP (GB)
(72) Inventor: The designation of the inventor has not yet been filed
(74) Representative: Mewburn Ellis LLP

(57) **Abstract**

A smoking substitute apparatus (150) comprising an air passage (170), a reservoir (160) formed from an air-permeable substrate and arranged to allow air to be drawn through the reservoir (160), the reservoir (160) being loaded with a source of nicotine, and one or more one-way valves (166) arranged along the air passage (170) and configured to allow air to flow along the air passage (170) in an upstream to downstream direction.

## Description

### Field of the Invention

The present invention relates to an aerosol delivery apparatus. Such an apparatus is of particular, but not necessary exclusive interest as a smoking substitute apparatus. It is a preferred feature of operation of the apparatus that it is able to deliver an active ingredient (such as nicotine) to a user for inhalation without producing a visible vapour cloud.

### Background

The smoking of tobacco is generally considered to expose a smoker to potentially harmful substances. It is thought that a significant amount of the potentially harmful substances are generated through the burning and/or combustion of the tobacco and the constituents of the burnt tobacco in the tobacco smoke itself.

Low temperature combustion of organic material such as tobacco is known to produce tar and other potentially harmful by-products. There have been proposed various smoking substitute systems in which the conventional smoking of tobacco is avoided.

Such smoking substitute systems can form part of nicotine replacement therapies aimed at people who wish to stop smoking and overcome a dependence on nicotine.

Known smoking substitute systems include electronic systems that permit a user to simulate the act of smoking by producing an aerosol (also referred to as a "vapour") that is drawn into the lungs through the mouth (inhaled) and then exhaled. The inhaled aerosol typically bears nicotine and/or a flavourant without, or with fewer of, the health risks associated with conventional smoking.

In general, smoking substitute systems are intended to provide a substitute for the rituals of smoking, whilst providing the user with a similar, or improved, experience and satisfaction to those experienced with conventional smoking and with combustible tobacco products.

The popularity and use of smoking substitute systems has grown rapidly in the past few years. Although originally marketed as an aid to assist habitual smokers wishing to quit tobacco smoking, consumers are increasingly viewing smoking substitute systems as desirable lifestyle accessories. There are a number of different categories of smoking substitute systems, each utilising a different smoking substitute approach. Some smoking substitute systems are designed to resemble a conventional cigarette and are cylindrical in form with a mouthpiece at one end. Other smoking substitute devices do not generally resemble a cigarette (for example, the smoking substitute device may have a generally box-like form, in whole or in part).

One approach is the so-called "vaping" approach, in which a vaporisable liquid, or an aerosol former, sometimes typically referred to herein as "e-liquid", is heated by a heating device (sometimes referred to herein as an electronic cigarette or "e-cigarette" device) to produce an aerosol vapour which is inhaled by a user. The e-liquid typically includes a base liquid, nicotine and may include a flavourant. The resulting vapour therefore also typically contains nicotine and/or a flavourant. The base liquid may include propylene glycol and/or vegetable glycerine.

A typical e-cigarette device includes a mouthpiece, a power source (typically a battery), a tank for containing e-liquid and a heating device. In use, electrical energy is supplied from the power source to the heating device, which heats the e-liquid to produce an aerosol (or "vapour") which is inhaled by a user through the mouthpiece.

E-cigarettes can be configured in a variety of ways. For example, there are "closed system" vaping smoking substitute systems, which typically have a sealed tank and heating element. The tank is prefilled with e-liquid and is not intended to be refilled by an end user. One subset of closed system vaping smoking substitute systems include a main body which includes the power source, wherein the main body is configured to be physically and electrically couplable to a consumable including the tank and the heating element. In this way, when the tank of a consumable has been emptied of e-liquid, that consumable is removed from the main body and disposed of. The main body can then be reused by connecting it to a new, replacement, consumable. Another subset of closed system vaping smoking substitute systems are completely disposable, and intended for one-use only.

There are also "open system" vaping smoking substitute systems which typically have a tank that is configured to be refilled by a user. In this way the entire device can be used multiple times.

An example vaping smoking substitute system is the myblu™ e-cigarette. The myblu™ e-cigarette is a closed system which includes a main body and a consumable. The main body and consumable are physically and electrically coupled together by pushing the consumable into the main body. The main body includes a rechargeable battery. The consumable includes a mouthpiece and a sealed tank which contains e-liquid. The consumable further includes a heater, which for this device is a heating filament coiled around a portion of a wick. The wick is partially immersed in the e-liquid, and conveys e-liquid from the tank to the heating filament. The system is controlled by a microprocessor on board the main body. The system includes a sensor for detecting when a user is inhaling through the mouthpiece, the microprocessor then activating the device in response. When the system is activated, electrical energy is supplied from the power source to the heating device, which heats e-liquid from the tank to produce a vapour which is inhaled by a user through the mouthpiece.

An alternative to vaping-type smoking substitute systems is an inhaler apparatus, of which a particular example is the Nicorette® inhalator (trade name). Such systems are often passive in the sense that they do not require a source of heat or other activation energy in order to generate a vapour. As with an e-cigarette, such an inhaler typically includes a mouthpiece and a main body containing a source of nicotine. In use, a user may inhale or "puff' on the mouthpiece to draw air over or through the nicotine source. The nicotine source may be, for example, an air-permeable substrate impregnated with nicotine. When the supply of nicotine in the nicotine source is depleted, such that the user no longer receives sufficient (or any) nicotine with each puff, the user can replace the nicotine source in order to continue nicotine delivery.

### Summary of the Invention

Smoking substitute systems (e.g. e-cigarettes) are generally regarded as having fewer of the health risks associated with conventional smoking, not only for the user themselves, but also for those nearby (i.e. those affected by passive smoking). However, the exhaling of a visible vapour cloud by the user may still be regarded in some circumstances as socially unacceptable, in light of the negative views surrounding smoking itself. Therefore, many locations where smoking is not permitted also do not allow smoking substitute systems, instead requiring users to use the same designated smoking areas as smokers themselves. This can reduce the attractiveness of smoking substitute systems, and reduce their uptake as a smoking cessation aid.

Accordingly, it would be advantageous to provide a smoking substitute system that can provide a similar user experience to an e-cigarette, but without producing a visible vapour cloud. Alternatively or additionally, it would be advantageous to provide a smoking substitute system that can allow a user to select whether or not the system produces a vapour cloud by switching between one system operating mode where a vapour cloud is produced and another system operating mode where a vapour cloud is not produced.

Still further, based on the insight of the present inventors, it would be advantageous to provide an aerosol delivery system, not necessarily limited to a smoking substitute system, for the delivery of an active ingredient to a user by inhalation, providing the beneficial effects referred to above.

The present disclosure has been devised in the light of the above considerations.

In a general aspect, the present invention relates to an aerosol delivery apparatus comprising an air permeable reservoir and at least one-way valve arranged in the air passage, the one or more one-way valves being provided to control air-flow through the air passage.

According to a first preferred aspect there is provided an aerosol delivery apparatus comprising an air passage, a reservoir formed from an air-permeable substrate and arranged to allow air to be drawn through the reservoir, the reservoir being loaded with a source of an active ingredient, and one or more one-way valves arranged along the air passage and configured to allow air to flow along the air passage in an upstream to downstream direction.

Providing at least one one-way valve in the air passage can reduce, substantially prevent or prevent air flow along the air passage in a downstream to upstream direction, controlling flow of both air and vapour through the passage and apparatus. Furthermore, the incorporation of a one-way valve may allow there to be a threshold pressure difference below which air flow will be blocked by the valve even in the upstream to downstream direction. This can ensure that the one-way valve is only opened for example when a user draws air through the apparatus. Such an arrangement can serve to prolong the useful lifetime of the reservoir.

In some embodiments, the aerosol delivery apparatus is a smoking substitute apparatus. In such embodiments, the active ingredient may comprise or consist of nicotine.

Optionally, at least one of the one or more one-way valves may be arranged upstream of the reservoir along the air passage.

Conveniently, at least one of the one or more one-way valves may be arranged downstream of the reservoir along the air passage.

Advantageously, the apparatus may comprise a first one-way valve and a second one-way valve arranged within the air passage, wherein the first one-way valve is arranged upstream of the reservoir along the air passage, and the second one-way valve is arranged downstream of the reservoir along the air passage.

Optionally, the aerosol delivery apparatus may further comprise a heater arranged in the air passage and upstream of the reservoir, the heater being operable to heat air passing through the air passage.

Conveniently, the heater may comprise an electrically heatable mesh.

Advantageously, the heater may comprise an electrically heatable heating coil.

Optionally, the heater may be heatable by resistive heating using an electrical current.

Conveniently, a one-way valve of the one-way valves may be arranged upstream of the heater along the air passage.

Advantageously, a one-way valve of the one-way valves may be a duckbill valve.

Optionally, a one-way valve of the one-way valves may be a ball one-way valve.

Conveniently, the ball one-way valve may further comprise a spring to locate the ball within the valve.

Advantageously, the combined resistance to draw presented by the one-way valve, reservoir and air passage may be substantially equal to that of a conventional cigarette.

Optionally, the reservoir is a consumable component of the aerosol delivery apparatus.

According to a second preferred aspect, the aerosol delivery apparatus is comprised by or within a cartridge configured for engagement with a base unit, the cartridge and base unit together forming a aerosol delivery system.

According to a third preferred aspect, there is provided an aerosol delivery system comprising a base unit, and an aerosol delivery apparatus according to the second aspect, wherein the aerosol delivery apparatus is removably engageable with the base unit.

According to a fourth preferred aspect, there is provided a method of using an aerosol delivery apparatus according to the first or second aspects to generate an aerosol.

The aerosol delivery apparatus may be in the form of a consumable. The consumable may be configured for engagement with a main body. When the consumable is engaged with the main body, the combination of the consumable and the main body may form an aerosol delivery system such as a closed aerosol delivery system. For example, the consumable may comprise components of the system that are disposable, and the main body may comprise non-disposable or non-consumable components (e.g. power supply, controller, sensor, etc.) that facilitate the generation and/or delivery of aerosol by the consumable. In such an embodiment, an aerosol precursor (e.g. e-liquid) and/or other nicotine source (e.g. nicotine-infused air-permeable substrate) may be replenished by replacing a used consumable with an unused consumable.

Alternatively, the aerosol delivery apparatus may be a non-consumable apparatus (e.g. that is in the form of an open aerosol delivery system). In such embodiments an aerosol former (e.g. e-liquid) of the system may be replenished by re-filling, e.g. a reservoir of the aerosol delivery apparatus, with the aerosol precursor (rather than replacing a consumable component of the apparatus).

In light of this, it should be appreciated that some of the features described herein as being part of the aerosol delivery apparatus may alternatively form part of a main body for engagement with the aerosol delivery apparatus. This may be the case in particular when the aerosol delivery apparatus is in the form of a consumable.

Where the aerosol delivery apparatus is in the form of a consumable, the main body and the consumable may be configured to be physically coupled together. For example, the consumable may be at least partially received in a recess of the main body, such that there is an interference fit between the main body and the consumable. Alternatively, the main body and the consumable may be physically coupled together by screwing one onto the other, or through a bayonet fitting, or the like.

Thus, the aerosol delivery apparatus may comprise one or more engagement portions for engaging with a main body. In this way, one end of the aerosol delivery apparatus may be coupled with the main body, whilst an opposing end of the aerosol delivery apparatus may define a mouthpiece of the aerosol delivery system.

In order to generate an aerosol, the reservoir comprises at least one volatile compound that is intended to be vaporised/aerosolised and that may provide the user with a recreational, wellness, nutritional, physiological and/or medicinal effect when inhaled. Such a volatile compound is referred to herein as an "active agent" or "active ingredient".

The active ingredient may comprise or consist of nicotine. However, in some embodiments, the active ingredient may not comprise nicotine, and may instead comprise or consist of one or more of a nutritional agent, a pharmaceutical agent or a flavour agent.

Suitable active agents include the group consisting of: nicotine, cocaine, caffeine (anhydrous or salts thereof), vitamins, minerals, amino acids, plant or herbal concentrated extracts, sugars, opiates and opioids, cathine and cathinone, kavalactones, mysticin, beta-carboline alkaloids, salvinorin A, cannabinoids, phytocannabinoids, one or more flavourants, together with any combinations, functional equivalents to, and/or synthetic alternatives of the foregoing.

Example flavourants may include menthol, liquorice, chocolate, fruit flavour (including e.g. citrus, cherry etc.), vanilla, spice (e.g. ginger, cinnamon) and tobacco flavour.

Cannabinoid compounds include phyto-cannabinoids which include:
- cannabidiol (CBD) and its derivatives/homologues (e.g. cannabidiol mono(m)ethyl ether, cannabidivarin (CBDV), cannabidiorcol, cannabidiolic acid, cannabidivarinic acid);
- cannabinodiol (CBND) and its derivatives/homologues (e.g. carrabinodivarin);
- cannabigerol (CBG) and its derivatives/homologues (e.g. cannabigerol mono(m)ethyl ether, cannabinerolic acid A, cannabigerovarin, cannabigerolic acid A, cannabigerolic acid A mono(m)ethyl ether, cannabigerovarinic acid A);
- cannabinol (CBN) and its derivatives/homologues (e.g. cannabivarin/cannabivarol (CBV), cannabiorcol, cannabinolic acid, cannabinol (m)ethyl ester);
- tetrahydrocannabinol (THC) and its derivatives/homologues (e.g. tetrahydrocannabivarin (THCV), tetrahydrocannabiorcol, tetrahydrocannabinolic acid A/B, tetrahydrocannabivarinic acid A, tetrahydrocannabiorcolic acid A/B, isotetrahydrocannabinol, isotetrahydrocannabivarin);
- cannabicyclol (CBL) and its derivatives/homologues (e.g. cannabicyclolic acid, cannabicyclovarin);
- cannabichromene (CBC) and its derivatives/homologues (e.g. cannabichromenic acid A, cannabichromevarin (CBCV), cannabichromevarinic acid A);
- cannabielsoin (CBE) and its derivatives/homologues (e.g. cannabielsoic acid A/B, cannabiglendol, dehydrocannabifuran, cannabifuran);
- cannabicitran (CBT) and its derivatives/homologues;
- cannabitriol and its derivatives/homologues (e.g. ethyl cannabitriol, dihydroxy-tetrahydrocannabinol, cannabidiolic acid A cannabitriol ester, dihydroxy-hexahydrocannabinol (cannabiripsol), cannabitetrol, oxo-tetrahydrocannabinol); and
- cannabichromanone (CBCN) and its derivatives/homologues (e.g. cannabicoumaronone).

In some embodiments, the cannabinoid compound is selected from at least one of cannabidiol (CBD) and its derivatives/homologues e.g. cannabiodiol-C₅ (CBD-C₅), cannabidiol-C₄ (CBD-C₄), cannabidiol mono(m)ethyl ether (CBDM-C₅), cannabidivarin (CBDV-C₃), cannabidiorcol (CBD-C₁), cannabidiolic acid (CBDA-C₅), cannabidivarinic acid (CBDVA-C₃).

In some embodiments, the cannabinoid compound is selected from at least one of tetrahydrocannabinol (THC) and its derivatives/homologues, e.g. Δ⁹-tetrahydrocannabinol (Δ⁹-THC-C₅ / *cis*-Δ⁹-THC-C₅), Δ⁸-tetrahydrocannabinol (Δ⁸-THC-C₅), Δ⁸-tetrahydrocannabinolic acid A (Δ⁸-THCA-C₅ A), Δ⁹-tetrahydrocannabinol-C₄ (Δ⁹-THC-C₄), Δ⁹-tetrahydrocannabivarin (Δ⁹-THCV-C₃), Δ⁹-tetrahydrocannabiorcol (Δ⁹-THCO-C₁), Δ⁹-tetrahydrocannabinolic acid A (Δ⁹-THCA-C₅A), Δ⁹-tetrahydrocannabinolic acid B (Δ⁹-THCA-C₅ B), Δ⁹-tetrahydrocannabinolic acid-C₄ A and/or B (Δ⁹-THCA-C₄A and/or B), Δ⁹-tetrahydrocannabivarinic acid A (Δ⁹-THCVA-C₃ A), Δ⁹-tetrahydrocannabiorcolic acid A and/or B (Δ⁹-THCOA-C₁ A and/or B), isotetrahydrocannabinol and isotetrahydrocannabivarin.

The total amount of cannabinoid compounds in the substrate may be at least 200 mg; for example, it may be at least 250 mg, at least 300 mg, at least 400 mg, at least 500 mg. In some cases, lower amounts may be preferred. The total amount of cannabinoid compounds in the substrate may therefore be at least 10 mg, at least 20 mg, at least 30 mg, at least 40 mg, at least 50 mg, at least 75 mg, at least 100 mg.

In some cases, it may be desirable to limited the total amount of cannabinoid compounds, which may be not more than 200 mg, not more than 175 mg, not more than 150 mg, not more than 125 mg, not more than 100 mg, not more than 75 mg, not more than 50 mg, not more than 40 mg, not more than 30 mg, not more than 20 mg, not more than 10 mg. In some cases, the total amount of the cannabinoid compounds may be not more than 5 mg.

Where THC is included, either as one cannabinoid compound in a mixture or as the only cannabinoid, the total of amount of THC may be limited. In some cases, the total amount of THC in the substrate is not more than 100 mg, not more than 75 mg, not more than 50 mg, not more than 40 mg, not more than 30 mg, not more than 20 mg, not more than 15 mg, not more than 10 mg, not more than 5 mg, not more than 3 mg. In some cases, the amount of THC may be 0.1 to 30 mg, for example 1 to 30 mg, for example 1 to 20 mg, for example 1 to 10 mg, for example 1 to 5 mg, for example 1 to 3 mg.

As already disclosed above, the aerosol delivery apparatus comprises a reservoir configured to store an aerosol precursor. The aerosol precursor may be formulated so as to produce a non-visible or substantially non-visible vapour. The aerosol precursor may comprise a base liquid. The aerosol precursor may additionally comprise nicotine. The aerosol precursor may be an e-liquid. The aerosol precursor may consist substantially of nicotine or a nicotine compound. The aerosol precursor may further comprise a flavourant. Alternatively, the aerosol precursor may be substantially flavourless. That is, the aerosol precursor may not contain any deliberately added additional flavourant. A flavourant may be provided as a separate flavourant aerosol precursor, such that the aerosol precursor and flavourant aerosol precursor may be separately vaporised to form an aerosol comprising both the aerosol precursor and the flavourant aerosol precursor.

The air-permeable substrate, storing the aerosol precursor and/or the flavourant, may constitute the entirety of the reservoir. The air-permeable substrate may be impregnated with the aerosol precursor and/or the flavourant aerosol precursor. The substrate material may be a foamed polymer which will allow for airflow to pass through the substrate at a given pressure drop value, so as to provide a comfortable 'draw' sensation for the user. The substrate may be, for example, a sintered polyethylene or a PET foam.

The substrate may be impregnated with nicotine via immersion in a liquid containing nicotine and a volatile carrier (for example a solution of nicotine in ethanol). The substrate may be immersed to evenly soak the substrate. Once removed and left to dry or baked in an oven, the carrier is evaporated and the nicotine is left evenly spread throughout the substrate.

The aerosol precursor and/or the flavourant aerosol precursor may be formulated to form a vapour when ambient air is drawn through the reservoir. Alternatively, the aerosol precursor and/or the flavourant aerosol precursor may be formulated to form a vapour when heated air is drawn through the reservoir. The reservoir may comprise a monolithic substrate. The reservoir may consist of a plurality of substrates, each arranged to allow air to be drawn therethrough, and each comprising one or both of an aerosol precursor and the flavourant aerosol precursor. The aerosol precursor and/or the flavourant aerosol precursor may be provided in spatially coterminous or spatially distinct regions of the reservoir.

The aerosol delivery apparatus may comprise more than one passage for fluid (e.g. air) flow therethrough. Where more than one passage is present, one or more of the passages may be distinct, such that there is no intersection between the passages. One or more of the passages may comprise junctions or openings therebetween such that fluid within the passages can mix within the smoking substitute apparatus.

The passage through the reservoir comprises one or more valves to control fluid flow. The valves include one or more one-way valve to ensure fluid (i.e. air) can only flow through the passage in a desired direction. Further valves may be provided that may be operable to open and close the passage such that fluid is enabled to or prevented from flowing through the passage. More than one such valve may be linked such that the valves may be operated in combination or in synchronism with each other. A valve to open and close a passage may be controlled by mechanical means (i.e. the user moves the valve using a control lever or similar) or by electrical control (i.e. moved in response to a control signal from a processor or control system of the aerosol delivery apparatus).

The passages may extend through (at least a portion of) the aerosol delivery apparatus, between openings that may define an inlet and an outlet of a passage. Each inlet and outlet may be in fluid communication with only one passage, or a subset of the passages, or all the passages in the aerosol delivery apparatus. The outlet or outlets may be at a mouthpiece of the aerosol delivery apparatus. In this respect, a user may draw fluid (e.g. air) into and through a passage by inhaling at the outlet (i.e. using the mouthpiece).

One or more of the fluid passages may comprise a heater for heating the fluid (i.e. air) passing through the passage. The heater may, for example, be arranged upstream of a reservoir formed from an air permeable substrate such that air warmed by the heater is drawn through the reservoir to enable or increase nicotine or flavourant vapourisation and subsequent entrainment in the air-flow. The heater to heat the air may comprise one or more meshes arranged within the fluid passage. The heater to heat the air may comprise one or more thermally conductive elements to conduct heat from a heater to the air passage or to increase the heat transfer between the heater and the air. Alternatively, the heat source may be non-electrical. For example, the heat to heat the air may be generated by an exothermic reaction. An exothermic reaction heat source may comprise a single-use (i.e. consumable) reaction container. Alternatively, an exothermic reaction heat source may be rechargeable (e.g. by using a reversible reaction such as a crystallisation process).

The aerosol delivery apparatus (or main body engaged with the aerosol delivery apparatus) may comprise a power source. The power source may be electrically connected (or connectable) to a heater of the aerosol delivery apparatus (e.g. when the aerosol delivery apparatus is engaged with the main body). The power source may be a battery (e.g. a rechargeable battery). A connector in the form of e.g. a USB port may be provided for recharging this battery.

When the aerosol delivery apparatus is in the form of a consumable, the aerosol delivery apparatus may comprise an electrical interface for interfacing with a corresponding electrical interface of the main body. One or both of the electrical interfaces may include one or more electrical contacts. Thus, when the main body is engaged with the consumable, the electrical interface of the main body may be configured to transfer electrical power from the power source to a heater of the consumable via the electrical interface of the consumable.

The electrical interface of the aerosol delivery apparatus may also be used to identify the aerosol delivery apparatus (in the form of a consumable) from a list of known types. For example, the consumable may have a certain concentration of nicotine and the electrical interface may be used to identify this. The electrical interface may additionally or alternatively be used to identify when a consumable is connected to the main body.

Again, where the aerosol delivery apparatus is in the form of a consumable, the main body may comprise an identification means, which may, for example, be in the form of an RFID reader, a barcode or QR code reader. This identification means may be able to identify a characteristic (e.g. a type) of a consumable engaged with the main body. In this respect, the consumable may include any one or more of an RFID chip, a barcode or QR code, or memory within which is an identifier and which can be interrogated via the identification means.

The aerosol delivery apparatus or main body may comprise a controller, which may include a microprocessor. The controller may be configured to control the supply of power from the power source to the heater(s) of the aerosol delivery apparatus (e.g. via the electrical contacts). A memory may be provided and may be operatively connected to the controller. The memory may include non-volatile memory. The memory may include instructions which, when implemented, cause the controller to perform certain tasks or steps of a method.

The main body or aerosol delivery apparatus may comprise a wireless interface, which may be configured to communicate wirelessly with another device, for example a mobile device, e.g. via Bluetooth®. To this end, the wireless interface could include a Bluetooth® antenna. Other wireless communication interfaces, e.g. WiFi®, are also possible. The wireless interface may also be configured to communicate wirelessly with a remote server.

A puff sensor may be provided that is configured to detect a puff (i.e. inhalation from a user). The puff sensor may be operatively connected to the controller so as to be able to provide a signal to the controller that is indicative of a puff state (i.e. puffing or not puffing). The puff sensor may, for example, be in the form of a pressure sensor or an acoustic sensor. That is, the controller may control power supply to the heater(s) of the consumable and/or aerosol delivery apparatus in response to a puff detection by the sensor. The control may be in the form of activation of the heater(s) in response to a detected puff. That is, the aerosol delivery apparatus may be configured to be activated when a puff is detected by the puff sensor. When the aerosol delivery apparatus is in the form of a consumable, the puff sensor may be provided in the consumable or alternatively may be provided in the main body. Where multiple independent passages are provided within the aerosol delivery apparatus, each of the passages may have a puff sensor.

The term "flavourant" is used to describe a compound or combination of compounds that provide flavour and/or aroma. For example, the flavourant may be configured to interact with a sensory receptor of a user (such as an olfactory or taste receptor). The flavourant may include one or more volatile substances.

The flavourant may be provided in solid or liquid form. The flavourant may be natural or synthetic. For example, the flavourant may include menthol, liquorice, chocolate, fruit flavour (including e.g. citrus, cherry etc.), vanilla, spice (e.g. ginger, cinnamon) and tobacco flavour. The flavourant may be evenly dispersed or may be provided in isolated locations and/or varying concentrations.

The first aerosol generator comprises an air-permeable substrate. This may comprise plant material. The plant material may comprise least one plant material selected from the list including *Amaranthus dubius, Arctostaphylos uva-ursi* (Bearberry), *Argemone mexicana, Amica, Artemisia vulgaris,* Yellow Tees, *Galea zacatechichi, Canavalia maritima* (Baybean), *Cecropia mexicana* (Guamura), *Cestrum noctumum, Cynoglossum virginianum* (wild comfrey), *Cytisus scoparius, Damiana, Entada rheedii, Eschscholzia califomica* (California Poppy), *Fittonia albivenis, Hippobroma longiflora, Humulus japonica* (Japanese Hops), *Humulus lupulus* (Hops), *Lactuca virosa* (Lettuce Opium), *Laggera alata, Leonotis leonurus, Leonurus cardiaca* (Motherwort), *Leonurus sibiricus* (Honeyweed), *Lobelia cardinalis, Lobelia inflata* (Indian-tobacco), *Lobelia siphilitica, Nepeta cataria* (Catnip), *Nicotiana species* (Tobacco), *Nymphaea alba* (White Lily), *Nymphaea caerulea* (Blue Lily), Opium poppy, *Passiflora incamata* (Passionflower), *Pedicularis densiflora* (Indian Warrior), *Pedicularis groenlandica* (Elephant's Head), *Salvia divinorum, Salvia dorrii* (Tobacco Sage), Salvia species (Sage), *Scutellaria galericulata, Scutellaria lateriflora, Scutellaria nana, Scutellaria* species (Skullcap), *Sida acuta* (Wireweed), *Sida rhombifolia, Silene capensis, Syzygium aromaticum* (Clove), *Tagetes lucida* (Mexican Tarragon), *Tarchonanthus camphoratus, Tumera diffusa* (Damiana), *Verbascum* (Mullein), *Zamia latifolia* (Maconha Brava) together with any combinations, functional equivalents to, and/or synthetic alternatives of the foregoing.

In some embodiments, the plant material is tobacco. Any type of tobacco may be used. This includes, but is not limited to, flue-cured tobacco, burley tobacco, Maryland Tobacco, dark-air cured tobacco, oriental tobacco, dark-fired tobacco, perique tobacco and rustica tobacco. This also includes blends of the above mentioned tobaccos.

Any suitable parts of the tobacco plant may be used. This includes leaves, stems, roots, bark, seeds and flowers.

The tobacco may comprise one or more of leaf tobacco, stem tobacco, tobacco powder, tobacco dust, tobacco derivatives, expanded tobacco, homogenised tobacco, shredded tobacco, extruded tobacco, cut rag tobacco and/or reconstituted tobacco (e.g. slurry recon or paper recon).

The air-permeable substrate may comprise a gathered sheet of homogenised (e.g. paper/slurry recon) tobacco or gathered shreds/strips formed from such a sheet.

In some embodiments, the sheet used to form the aerosol-forming substrate has a grammage greater than or equal to 100 g/m², e.g. greater than or equal to 110 g/m² such as greater than or equal to 120 g/m².

The sheet may have a grammage of less than or equal to 300 g/m² e.g. less than or equal to 250 g/m² or less than or equal to 200 g/m².

The sheet may have a grammage of between 120 and 190 g/m².

The air-permeable substrate may comprise at least 50 wt% plant material, e.g. at least 60 wt% plant material e.g. around 65 wt% plant material. The air-permeable substrate may comprise 80 wt% or less plant material e.g. 75 or 70 wt% or less plant material.

The air-permeable substrate may comprise one or more additives selected from flavourants, fillers and binders.

Typically, the air-permeable substrate does not comprise a humectant. Humectants may be provided in heat not burn (HNB) tobacco charges. In such cases, humectants are provided as vapour generators, the generated vapour being used to help carry volatile active compounds and to increase visible vapour. Accordingly, it is preferred that the air-permeable substrate does not comprise one or more humectants such as polyhydric alcohols (e.g. propylene glycol (PG), triethylene glycol, 1,2-butane diol and vegetable glycerine (VG)) and their esters (e.g. glycerol mono-, di- or tri-acetate). If such humectants are present in the air-permeable substrate, they may be present at a low level, such as less than 0.5 wt%, more preferably less than 0.1 wt%.

Suitable binders are known in the art and may act to bind together the components forming the air-permeable substrate. Binders may comprise starches and/or cellulosic binders such as methyl cellulose, ethyl cellulose, hydroxypropyl cellulose, hydroxyethyl cellulose and methyl cellulose, gums such as xanthan, guar, arabic and/or locust bean gum, organic acids and their salts such as alginic acid/ sodium alginate, agar and pectins.

Preferably the binder content is 5 to 10 wt% of the air-permeable substrate e.g. around 6 to 8 wt%.

Suitable fillers are known in the art and may act to strengthen the air-permeable substrate. Fillers may comprise fibrous (non-tobacco) fillers such as cellulose fibres, lignocellulose fibres (e.g. wood fibres), jute fibres and combinations thereof.

Preferably, the filler content is 5 to 10 wt% of the aerosol-forming substrate e.g. around 6 to 9 wt%.

The air-permeable substrate may comprise an aqueous and/or non-aqueous solvent. In some embodiments, the air-permeable substrate has a water content of between 4 and 10 wt% e.g. between 6-9 wt% such as between 7-9 wt%. Such low moisture content in the air-permeable substrate typically has the effect that, when the air-permeable substrate is exposed to heated air, there would typically not be produced a substantial visible vapour. It is to be noted that in one embodiment it is possible to use as the air-permeable substrate a low moisture tobacco material with its natural nicotine content. The natural nicotine content then meets the requirements of the active agent.

The air-permeable substrate may be at least partly circumscribed by a wrapping layer e.g. a paper wrapping layer. The wrapping layer may overlie an inner foil layer or may comprise a paper/foil laminate (with the foil innermost).

The plant material may comprise cannabis plant material including *Cannabis sativa, Cannabis indica* and *Cannabis rudealis.* The plant material may comprise *Echinacea purpurea, Echinacea angustifolia, Acmella oleracea, Helichrysum umbraculigerum,* or *Radula marginata.* This also includes blends of the above mentioned plant material.

In some embodiments, the cannabinoid-containing plant material is cannabis. The plant may be a traditional strain, or may be a strain bred or other modified (e.g. genetically) to produce certain levels of some cannabinoids compounds, e.g. low levels of THC or high levels of THC.

Any suitable parts of the cannabinoid-containing plant may be used. Thus the cannabinoid-containing plant material may comprise leaves, stems, roots, bark, seeds, buds and flowers (which may be cured).

The invention includes the combination of the aspects and preferred features described except where such a combination is clearly impermissible or expressly avoided.

### Summary of the Figures

So that the invention may be understood, and so that further aspects and features thereof may be appreciated, embodiments illustrating the principles of the invention will now be discussed in further detail with reference to the accompanying figures, in which:
Figure 1 is a schematic front view of a smoking substitute system, according to a first embodiment, in an engaged position;
Figure 2 is a schematic front view of the smoking substitute system of the first embodiment in a disengaged position;
Figure 3 is a schematic longitudinal cross sectional view of a smoking substitute apparatus of the first embodiment;
Figure 4A is a schematic longitudinal cross sectional view of a duckbill one-way valve in a closed position;
Figure 4B is a schematic longitudinal cross sectional view of a duckbill one-way valve in an open position;
Figure 5A is a schematic longitudinal cross sectional view of a ball one-way valve in a closed position; and
Figure 5B is a schematic longitudinal cross sectional view of a ball one-way valve in an open position.

### Detailed Description of the Invention

Further background to the present invention and further aspects and embodiments of the present invention will now be discussed with reference to the accompanying figures. Further aspects and embodiments will be apparent to those skilled in the art. The contents of all documents mentioned in this text are incorporated herein by reference in their entirety.

The embodiments of the invention are described as smoking substitute apparatuses or systems, in which the active ingredient typically comprises or consists of nicotine. However, on the basis of the present disclosure it will be apparent that the invention can be embodied more generally as an aerosol delivery apparatus or system. In such aerosol delivery apparatuses or systems the active ingredient may not comprise nicotine, and may instead comprise or consist of one or more of a nutritional agent, a pharmaceutical agent or a flavour agent.

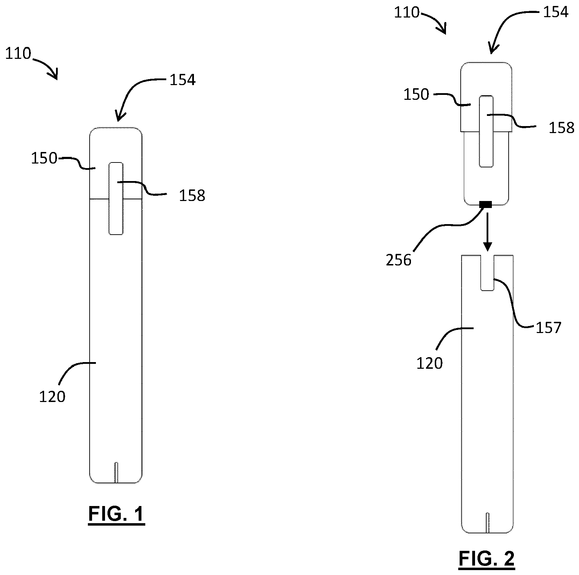

Figures 1 and 2 illustrate a smoking substitute system in the form of an e-cigarette system 110. The system 110 comprises a main body 120 of the system 110, and a smoking substitute apparatus in the form of an e-cigarette consumable (or "pod") 150. In the illustrated embodiment the consumable 150 (sometimes referred to herein as a smoking substitute apparatus) is removable from the main body 120, so as to be a replaceable component of the system 110. The e-cigarette system 110 is a closed system in the sense that it is not intended that the consumable should be refillable with nicotine-based liquid by a user.

As is apparent from Figures 1 and 2, the consumable 150 is configured to engage the main body 120. Figure 1 shows the main body 120 and the consumable 150 in an engaged state, whilst Figure 2 shows the main body 120 and the consumable 150 in a disengaged state. When engaged, a portion of the consumable 150 is received in a cavity of corresponding shape in the main body 120 and is retained in the engaged position by way of a snap-engagement mechanism. In other embodiments, the main body 120 and consumable 150 may be engaged by screwing one into (or onto) the other, or through a bayonet fitting, or by way of an interference fit. In the illustrated embodiment, the consumable 150 is a "single-use" consumable 150. The term "single-use" does not necessarily mean the consumable is designed to be disposed of after a single smoking session. Rather, it defines that the consumable 150 is not designed to be refilled, and is instead disposed of and replaced after usage.

The power source of the main body 120 may be in the form of a battery (e.g. a rechargeable battery such as a lithium ion battery). The main body 120 may comprise a connector in the form of e.g. a USB port for recharging this battery. The main body 120 may also comprise a controller that controls the supply of power from the power source to the main body electrical contacts (and thus to the heater 164). That is, the controller may be configured to control a voltage applied across the main body electrical contacts, and thus the voltage applied across the heater 164. In this way, the heater 164 may only be heated under certain conditions (e.g. during a puff and/or only when the system is in an active state). In this respect, the main body 120 may include a puff sensor (not shown) that is configured to detect a puff (i.e. inhalation). The puff sensor may be operatively connected to the controller so as to be able to provide a signal, to the controller, which is indicative of a puff state (i.e. puffing or not puffing). The puff sensor may, for example, be in the form of a pressure sensor or an acoustic sensor.

Although not shown, the main body 120 and consumable 150 may comprise a further interface which may, for example, be in the form of an RFID reader, a barcode or QR code reader. This interface may be able to identify a characteristic (e.g. a type) of a consumable 150 engaged with the main body 120. In this respect, the consumable 150 may include any one or more of an RFID chip, a barcode or QR code, or memory within which is an identifier and which can be interrogated via the interface.

The system 110 is configured to vaporise an aerosol precursor, which in the illustrated embodiment is a nicotine-based liquid impregnated into a substrate 160. When air is drawn through or over the nicotine-impregnated substrate 160, the nicotine is vaporised and entrained in the airflow to thereby be delivered to a user. The vapour or aerosol produced by the aerosol generator is less visible than that produced by a conventional e-liquid from an e-cigarette when exhaled by a user. Preferably, the vapour or aerosol generated by the aerosol generator is invisible or substantially invisible when exhaled by a user. The porosity or air permeability of at least part of the substrate 160 may be selected so as to provide a resistance to draw to a user that is comparable to a conventional cigarette.

A substrate 160 may be impregnated with nicotine by immersion in a solution of nicotine in a volatile carrier solvent (e.g. ethanol) such that the substrate 160 is evenly soaked. The substrate 160 can then be removed and left to dry or baked in an oven, meaning that the carrier is evaporated and the nicotine is left evenly spread throughout the substrate. The nicotine solution may further comprise a flavourant. Alternatively, a flavourant solution and a nicotine solution may be separately impregnated into the substrate 160.

Further details are now set out relating to the air-permeable substrate and its impregnation with an active ingredient.

Suitable materials and methods for manufacturing air permeable substrates are disclosed, for example, in US4800903, US4284089, US4813437, and US5167242, the entire contents of which are incorporated herein by reference.

US4800903 discloses that preferred materials for a polymeric plug are olefinic polymers, and preferably polyethylene or polypropylene, most preferably high density polyethylene. Use of high density polyethylene is preferred over, for example, amorphous polyethylene, since it provides a balance between ease of manufacturing and capacity for reversible nicotine absorption.

Meanwhile, some polymers are considered to be inherently unsuitable for use as an air permeable substrate. For example, some polymeric substances such as polystyrene and polycarbonate are dissolved by nicotine, rendering them unsuitable for forming a nicotine impregnated substrate. Furthermore, polymers containing halogens or nitrogen or sulphur are undesirable since they can produce noxious fumes.

To improve user satisfaction, it may be preferable to use an air permeable substrate that provides an equivalent resistance to draw to that of a conventional cigarette. For example, US4284089 discloses that a non-combustible cigarette with a draw resistance approximating that of a conventional cigarette would permit about 35 millilitres of air to be drawn through it during a 2 second period.

A substrate may be impregnated with nicotine by a variety of methods. For example, US4800903 indicates that liquid nicotine, nicotine vapour or a solution of nicotine may be used, and suggests that a solution of nicotine in supercritical liquid carbon dioxide may advantageously be used to impregnate the substrate. Alternatively, the substrate may be impregnated with nicotine via immersion in a liquid containing nicotine and a volatile carrier (for example a solution of nicotine in ethanol). The substrate is immersed to evenly soak the substrate. Once the substrate is removed from the liquid it can be left to dry or baked in an oven, evaporating away the carrier so that the nicotine is left evenly distributed throughout the substrate.

US5167242 discloses that a polyethylene plug can be charged or loaded with a mixture of nicotine, menthol and ethanol in a weight ratio (nicotine:menthol:ethanol) of about 10:1:120 or 10:1:160. The menthol and nicotine are sequentially added to the ethanol in a mixing vessel to produce a solution. Meanwhile, the plugs are placed in a vacuum dryer, which is partially evacuated to create a lower internal pressure than that of the mixing vessel, allowing the nicotine/ethanol/methanol solution to be sucked into the vacuum dryer. The plugs remain immersed in the solution within the vacuum dryer for 10 minutes, after which the temperature is raised and the vacuum pump is started to evaporate the ethanol. The vacuum dryer is then filled with nitrogen, and a nitrogen atmosphere is maintained for the remainder of the packaging procedure to prevent oxygen contamination of the nicotine.

In alternative embodiments, the air-permeable substrate may be formed in a different manner. For example, the air-permeable substrate may be formed from tobacco. The tobacco may be leaf tobacco, tobacco derivatives, expanded tobacco, shredded tobacco, reconstituted tobacco or tobacco substitutes. Preferably the tobacco has a relatively low moisture content, for example less than 10wt% moisture. A typical minimum moisture content for the tobacco is not less than 4wt% moisture. Such low moisture content tobacco, when exposed to heated air, would typically not produce a substantial vapour. Accordingly, such an air-permeable substrate may be loaded with a source of an active ingredient, as described above.

Where the air-permeable substrate is formed for example of tobacco, the active agent may be applied to the air-permeable substrate by mixing and/or dissolving the active agent in a suitable carrier liquid such as a solvent (e.g. water, ethanol, PG, glycerine, macrogol, caster oil, paraffin, (and derivatives thereof)). The air is typically heated to a suitable temperature. This temperature may be at least 30°C. This is in order to promote vaporisation of the active ingredient. The temperature is typically not greater than 80°C, or typically not greater than 70°C. This is in order to promote user comfort. It may also reduce or prevent the degradation of the air-permeable substrate and/or the active ingredient.

The nicotine-impregnated substrate 160 may be provided within a consumable 150. In such an embodiment, when the supply of nicotine in the nicotine-impregnated substrate 160 is depleted, the consumable 150 may be replaced. In other embodiments, the nicotine-impregnated substrate 160 itself may be a consumable component of the system 110. For example, the nicotine-impregnated substrate may be locatable within and removable from the system 110.

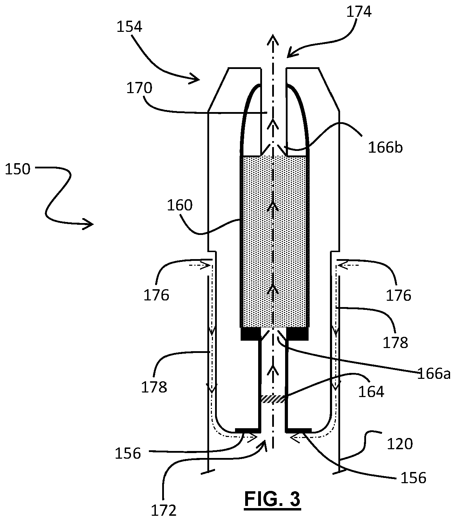

Figure 3 shows a schematic longitudinal cross-sectional view of the aerosol generator which forms part of the smoking substitute system shown in Figures 1 and 2. In Figure 3, the nicotine-impregnated substrate 160 is arranged within a passage 170. The passage 170 extends between an aerosol generator inlet 172 and an aerosol generator outlet 174 at opposing ends of the consumable 150. In this respect, the passage 170 comprises an upstream end at the end of the consumable 150 that engages with the main body 120, and a downstream end at an opposing end of the consumable 150 that comprises a mouthpiece 154 of the system 110.

When the consumable 150 is received in the cavity of the main body 120 as shown in Figure 3, a plurality of device air inlets 176 are formed at the boundary between the casing of the consumable and the casing of the main body. The device air inlets 176 are in fluid communication with the aerosol generator inlet 172 through an inlet flow channel 178 formed in the cavity of the main body which is of corresponding shape to receive a part of the consumable 150a. Air from outside of the system 110 can therefore be drawn into the passage 170 through the device air inlets 176 and the inlet flow channels 178.

When the consumable 150 is engaged with the main body 120, a user can inhale (i.e. take a puff) via the mouthpiece 154 so as to draw air through the passage 170, and so as to form an airflow (indicated by the dashed arrows in Figure 3) in a direction from the aerosol generator inlet 172 to the aerosol generator outlet 174. Air is thereby drawn through and/or around the nicotine-impregnated substrate 160, such that nicotine from the nicotine-impregnated substrate 160 can be entrained in the airflow.

In this embodiment, a heater 164 is arranged upstream of the nicotine-impregnated substrate 160 along the passageway 170. The heater 164 is operable to heat air passing through the passageway 170 to enable or enhance nicotine entrainment. In this embodiment, the heater 164 comprises an electrically heatable mesh located in the airflow stream. The mesh may be formed of a material that is heatable by resistive heating using an electrical current. In other embodiments, the heater 164 may comprise a plurality of meshes. The heater 164 may be located in a passage in the base unit 120 (not illustrated), with the base unit passageway being in fluid communication with the passageway 170 of the consumable 150a. In further embodiments, the heater 164 may be located externally of the passageway 170 but placed in thermal communication with the airflow via thermally conductive elements which extend into or across the passageway 170 (not illustrated). In still further embodiments, the heater may be omitted.

When the consumable 150 is engaged with the main body 120, electrical contacts 156 make contact with corresponding electrical contacts (not shown) of the main body 120. The main body electrical contacts are electrically connectable to a power source (not shown) of the main body 120, such that (in the engaged position) the heater 164 is electrically connectable to the power source. In this way, power can be supplied by the main body 120 to the heater 164 in order to heat the heater 164.

The passageway 170 may comprise one or more one-way valves 166. The one or more valves serve to control airflow through the passageway 170. The one or more valves are provided to prevent, substantially prevent or reduce airflow from a downstream to upstream direction along the passageway 170. A one-way valve may also be referred to as a check valve. In Figure 3, two valves 166 are located immediately upstream and immediately downstream, respectively, of the nicotine-impregnated substrate 160. In some embodiments, only one valve 166 may be provided. In further embodiments, a valve 166 may be located upstream of the heater 164.

Providing valves 166 immediately upstream and/or immediately downstream of the nicotine-impregnated substrate 160 may be advantageous for preventing nicotine or nicotine infused liquid from escaping from the nicotine-impregnated substrate 160 when the smoking substitute apparatus 150 is not in use. Providing a valve 166 upstream of a heater 164 allows control over the airflow, while not requiring the valve to be suitable to operate with heated air. A valve 166 provided upstream of a heater 164 may also improve the efficiency of utilisation for the heater, since heated air is prevented from flowing in an upstream direction from the heater 164.

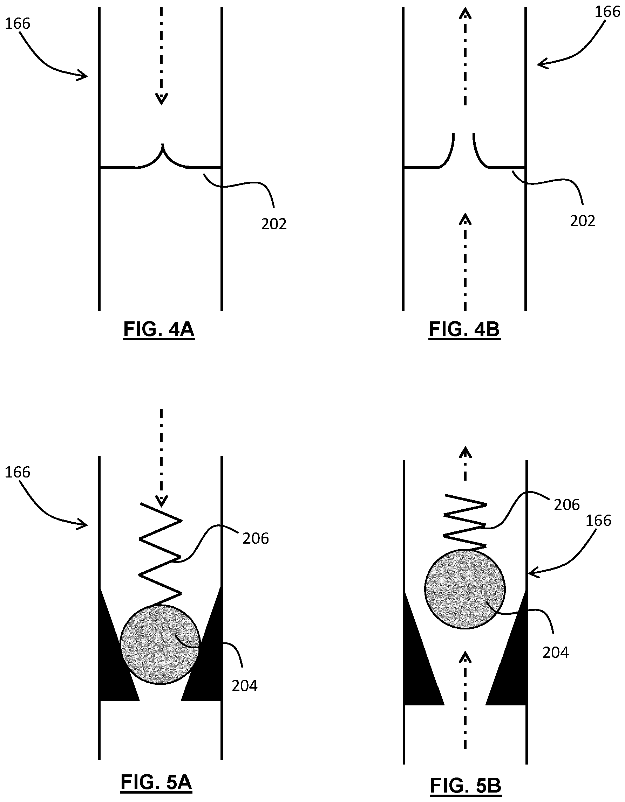

At least one of the one-way valves 166 may be a duckbill valve, as illustrated exemplarily in Figures 4A, 4B. A duckbill valve comprises an elastomeric material diaphragm 202 in which a portion is shaped like the bill or beak of a duck, comprising an opening or aperture therethrough. Fluid flow in the "allowed" direction opens the duckbill shaped portion (Fig. 4B), while fluid flow in a reverse direction (or absence of any fluid flow) cases the duckbill shaped portion to close (Fig. 4A). A pressure differential across the valve 166 from the upstream to downstream side may be required to open the valve and allow fluid to flow.

At least one of the one-way valves 166 may be a ball one-way valve, as illustrated exemplarily in Figures 5A and 5B. A ball one-way valve comprises a ball 204 located in a tapered passage so as to prevent fluid flow in a reverse direction (Figure 5A). Fluid flow in an allowed direction moves the ball 204, allowing flow around the ball 204 (Figure 5B). A ball one-way valve may further comprise a spring 206 to position the ball 204 within the valve. In some embodiments, the spring 206 may be omitted. As with the duckbill valve, a one-way ball valve may require a pressure differential across the valve 166 in an upstream to downstream direction to open the valve 166 and allow flow through the valve 166, particularly in valves where a spring 206 is present.

The pressure differential required to open a duckbill or ball one-way valve may therefore create a resistance to draw within the passage 170 of the smoking substitute apparatus 160. The one-way valve or valves may be configured such that, in combination with the reservoir 160, the resistance to draw of the smoking substitute apparatus as a whole is comparable to that of a conventional cigarette.

The features disclosed in the foregoing description, or in the following claims, or in the accompanying drawings, expressed in their specific forms or in terms of a means for performing the disclosed function, or a method or process for obtaining the disclosed results, as appropriate, may, separately, or in any combination of such features, be utilised for realising the invention in diverse forms thereof.

While the invention has been described in conjunction with the exemplary embodiments described above, many equivalent modifications and variations will be apparent to those skilled in the art when given this disclosure. Accordingly, the exemplary embodiments of the invention set forth above are considered to be illustrative and not limiting. Various changes to the described embodiments may be made without departing from the spirit and scope of the invention.

For the avoidance of any doubt, any theoretical explanations provided herein are provided for the purposes of improving the understanding of a reader. The inventors do not wish to be bound by any of these theoretical explanations.

Any section headings used herein are for organizational purposes only and are not to be construed as limiting the subject matter described.

Throughout this specification, including the claims which follow, unless the context requires otherwise, the words "have", "comprise", and "include", and variations such as "having", "comprises", "comprising", and "including" will be understood to imply the inclusion of a stated integer or step or group of integers or steps but not the exclusion of any other integer or step or group of integers or steps.

It must be noted that, as used in the specification and the appended claims, the singular forms "a," "an," and "the" include plural referents unless the context clearly dictates otherwise. Ranges may be expressed herein as from "about" one particular value, and/or to "about" another particular value. When such a range is expressed, another embodiment includes from the one particular value and/or to the other particular value. Similarly, when values are expressed as approximations, by the use of the antecedent "about," it will be understood that the particular value forms another embodiment. The term "about" in relation to a numerical value is optional and means, for example, +/- 10%.

The words "preferred" and "preferably" are used herein refer to embodiments of the invention that may provide certain benefits under some circumstances. It is to be appreciated, however, that other embodiments may also be preferred under the same or different circumstances. The recitation of one or more preferred embodiments therefore does not mean or imply that other embodiments are not useful, and is not intended to exclude other embodiments from the scope of the disclosure, or from the scope of the claims.

## Claims

1. An aerosol delivery apparatus (150) comprising:
an air passage (170);
a reservoir (160) formed from an air-permeable substrate and arranged to allow air to be drawn through the reservoir (160), the reservoir (160) being loaded with a source of an active ingredient; and
one or more one-way valves (166) arranged along the air passage (170) and configured to allow air to flow along the air passage (170) in an upstream to downstream direction.

2. An aerosol delivery apparatus (150) according to claim 1, wherein at least one of the one or more one-way valves (166) is:
arranged upstream of the reservoir (160) along the air passage (170); or
arranged downstream of the reservoir (160) along the air passage (170).

3. An aerosol delivery apparatus (150) according to either of claims 1 or 2, wherein:
the apparatus (160) comprises a first one-way valve (166a) and a second one-way valve (166b) arranged within the air passage (170), and wherein
the first one-way valve (166a) is arranged upstream of the reservoir along the air passage (170); and
the second one-way valve (166b) is arranged downstream of the reservoir along the air passage (170).

4. An aerosol delivery apparatus (150) according to any preceding claim, further comprising:
a heater (164) arranged in the air passage (170) and upstream of the reservoir, the heater (164) being operable to heat air passing through the air passage (170).

5. An aerosol delivery apparatus (150) according to claim 4, wherein:
the heater (164) comprises an electrically heatable mesh.

6. An aerosol delivery apparatus (150) according to any one of claims 4 to 5, wherein:
the heater (164) is heatable by resistive heating using an electrical current.

7. An aerosol delivery apparatus (160) according to any of claims 4 to 6, wherein:
a one-way valve (166) of the one-way valves (166) is arranged upstream of the heater (164) along the air passage (170).

8. An aerosol delivery apparatus (150) according to any preceding claim, wherein:
a one-way valve (166) of the one-way valves (166) is a duckbill valve.

9. An aerosol delivery apparatus (150) according to any preceding claim, wherein:
a one-way valve (166) of the one-way valves (166) is a ball one-way valve.

10. An aerosol delivery apparatus (150) according to claim 9, wherein:
the ball one-way valve (166) further comprises a spring (206) to locate the ball within the valve (166).

11. An aerosol delivery apparatus (150) according to any preceding claim, wherein:
the combined resistance to draw presented by the one-way valve (166), reservoir (160) and air passage (170) is substantially equal to that of a conventional cigarette.

12. An aerosol delivery apparatus (150) according to any preceding claim, wherein:
the reservoir (160) is a consumable component of the aerosol delivery apparatus (150).

13. An aerosol delivery apparatus (150) according to any one of claims 1 to 12, wherein the aerosol delivery apparatus (150) is comprised by or within a cartridge configured for engagement with a base unit (120), the cartridge and base unit together forming an aerosol delivery system (110).

14. An aerosol delivery system (110) comprising:
a base unit (120), and
an aerosol delivery apparatus (150) according to claim 13, wherein the aerosol delivery apparatus (150) is removably engageable with the base unit (120).

15. A method of using an aerosol delivery apparatus (150) according to any one of claims 1 to 13 to generate an aerosol.
